# EUROPEAN PATENT SPECIFICATION

(11) **EP 3 204 020 B1**
(45) Date of publication and mention of the grant of the patent: **18.01.2023**
(21) Application number: 15782170.3
(22) Date of filing: 02.10.2015
(51) Int. Cl.: A61K 35/17, C12N 5/0783

(54) **TREATING VIRAL HEMORRHAGIC FEVER WITH NK-92 CELLS**
BEHANDLUNG VON VIRALEM HÄMORRHAGISCHEM FIEBER MIT NK-92-ZELLEN
TRAITEMENT DE LA FIÈVRE HÉMORRAGIQUE VIRALE AVEC DES CELLULES NK-92

(30) Priority: 06.10.2014 US 201462060468 P; 27.03.2015 US 201562139421 P
(43) Date of publication of application: 16.08.2017
(73) Proprietor: ImmunityBio, Inc., Culver City, CA 90232 (US)
(72) Inventor: LEE, Tien, Culver City, CA 90232 (US); SIMON, Barry, Culver City, CA 90232 (US)
(74) Representative: Elkington and Fife LLP
(86) International application number: PCT/US2015/053862
(87) International publication number: WO 2016/057345

(56) References cited:
- WO-A2-2006/023148
- US-A1- 2013 295 044
- Y.K. TAM ET AL: "Characterization of Genetically Altered, Interleukin 2-Independent Natural Killer Cell Lines Suitable for Adoptive Cellular Immunotherapy", HUMAN GENE THERAPY, vol. 10, no. 8, 20 May 1999 (1999-05-20), pages 1359-1373, XP055233069, US ISSN: 1043-0342, DOI: 10.1089/10430349950018030
- GONG J-H ET AL: "CHARACTERIZATION OF A HUMAN CELL LINE (NK-92) WITH PEHNOTYPICAL AND FUNCTIONAL CHARACTERISTICS OF ACTIVATED NATURAL KILLER CELLS", LEUKEMIA, MACMILLAN PRESS LTD, US, vol. 8, no. 4, 1 April 1994 (1994-04-01), pages 652-658, XP002914219, ISSN: 0887-6924 cited in the application
- XIANGGUO QIU ET AL: "Reversion of advanced Ebola virus disease in nonhuman primates with ZMapp", NATURE, 29 August 2014 (2014-08-29), XP055234136, United Kingdom ISSN: 0028-0836, DOI: 10.1038/nature13777 cited in the application
- K. L. WARFIELD: "Role of Natural Killer Cells in Innate Protection against Lethal Ebola Virus Infection", THE JOURNAL OF EXPERIMENTAL MEDICINE, vol. 200, no. 2, 12 July 2004 (2004-07-12) , pages 169-179, XP055234045, US ISSN: 0022-1007, DOI: 10.1084/jem.20032141

## Description

Natural killer (NK) cells are cytotoxic lymphocytes that constitute a major component of the innate immune system. Natural killer (NK) cells, generally representing about 10-15% of circulating lymphocytes, bind and kill targeted cells, including virus-infected cells and many malignant cells, non-specifically with regard to antigen and without prior immune sensitization. Herberman et al., Science 214:24 (1981). Killing of targeted cells occurs by inducing cell lysis. NK cells used for this purpose are isolated from the peripheral blood lymphocyte ("PBL") fraction of blood from the subject, expanded in cell culture in order to obtain sufficient numbers of cells, and then re-infused into the subject. NK cells have been shown to be somewhat effective in both *ex vivo* therapy and *in vivo* treatment. However, such therapy is complicated by the fact that not all NK cells are cytolytic and the therapy is specific to the treated patient.

US 2013/295044 A1 describes a specialized subpopulation of natural killer cells that have enhanced effector functions and the potential to kill malignant tumor cells or infected cells when the natural killer cells are exposed to an antibody bound to the tumor cells or the infected cells.
NK-92 cells have previously been evaluated as a therapeutic agent in the treatment of certain cancers. Unlike NK cells, NK-92 is a cytolytic cancer cell line which was discovered in the blood of a subject suffering from a non-Hodgkins lymphoma and then immortalized *ex vivo.* NK-92 cells lack the major inhibitory receptors that are displayed by normal NK cells, but retain the majority of the activating receptors. Characterization of the NK-92 cell line is disclosed by Gong *et al*., 1994; and Yan *et al*., 1998. NK-92 cells do not, however, attack normal cells nor do they elicit an unacceptable immune rejection response in humans.

Viral hemorrhagic fever (VHF) is a group of diseases that are characterized by fevers and increased susceptibility to bleeding. VHF can be caused by several families of RNA viruses: *Arenaviridae, Filoviridae, Bunyaviridae, Flaviviridae,* and *Rhabdoviridae.* Severity of the diseases caused by each virus ranges from mild to severe. There is evidence that the Type I interferon (IFN-I) pathway enhances the mortality rate of VHF by contributing to the hemorrhagic effect of the virus (*i.e.*, making the blood vessels "leaky"). This pathway is mediated by CD8+ T-cells. *See,* Baccala, et al., PNAS 111(24): 8925-8930 (2014).

Ribavirin, an anti-viral drug, has shown some effectiveness against Lassa fever or hemorrhagic fever with renal syndrome. Some patients with Argentine hemorrhagic fever have been successfully treated with convalescent-phase plasma. However, these therapies are investigational, and there are no accepted treatments or vaccines for most of the viruses that cause VHF.

Recently, experimental antibodies have been used against *Filoviridae* viral infections. *See*, *e.g.,* Maruyama, et al., The Journal of Infectious Diseases 179(Suppl 1):S235-9 (1999); PCT Publication No. WO 2013/095738; Qiu X et al, Nature 514(7520):47-53 (2014); and Qiu X et al., Sci Rep 3:3365 (2013). However, these therapies are investigational, and given that the mortality of such VHF infections is not 100%, it is unknown whether these experimental therapies were successful or the patients recovered on their own. Presently there are no known treatments or vaccines for most of the viruses that cause VHF.

There thus remains a need for a successful treatment for VHF, and particularly severe, deadly VHF *(e.g.,* Ebola, Marburg, dengue fever, yellow fever, and the like).

### SUMMARY OF THE INVENTION

The invention is defined by the claims and provides NK-92 cells for use in a method for treating viral hemorrhagic fever (VHF) caused by Ebola virus, the method comprising administering the NK-92 cells to a patient who has or is suspected of having viral hemorrhagic fever, wherein the NK-92 cells are from American Type Culture Collection Accession No. CRL-2407 or CRL-2408 and wherein the NK-92 cells have not been modified to express an Fc receptor and display it on the cell surface.

Without being bound by theory, it is believed that the NK-92 cells target viral-infected cells.
Any references in the description to methods of treatment refer to the compounds, pharmaceutical compositions and medicaments of the present invention for use in a method for treatment of the human (or animal) body by therapy.

The viruses that cause VHF are difficult to treat or prevent. To date, few vaccines or therapies exist that are effective against VHF-causing viruses. The critical need for such therapies is seen by the recent rapid and unprecedented spread of Ebola in West Africa. Experimental antibody therapy (e.g., ZMapp, convalescent serum) has had some effect on the virus in isolated cases, but no therapies have been analyzed in clinical trials. Further, VHF represents a unique class of RNA viruses that are unpredictable and have great potential for mutation. To date, NK-92 cells have not been used in patients for treating viruses and have been limited to treating cancers. While NK cells are believed to treat viral infections, NK cells are often a target of such viruses, compromising the ability of NK cells to target the virus. Moreover, given the inability of the art to find a treatment for the VHF infections, it was not clear that NK-92 cells would be suitable for use in treating such infections. NK-92 cells have significant aberrations compared to naturally-occurring NK cells.

The present disclosure is predicated, in part, on the idea that NK-92 cells can target cells that have been infected by a virus that causes VHF. This disclosure is further predicated on the discovery that at least a subset of VHF, particularly Marburg virus and Ebola virus, deplete the natural killer cell population in an infected patient, possibly *via* targeting of CD-16-expressing cells. The immune system of a patient is thus compromised early in the infection, allowing the virus to proliferate rapidly. Without being bound by theory, it is believed that administration of NK-92 cells to the patient at any time during infection enhances the immune response. Such an enhancement allows the NK-92 cells to kill infected cells and virus in the body. The NK-92 cells would further provide a boost to allow the innate immune response to catch up and eventually clear the virus. NK-92 cells are particularly suited to this task because, unlike endogenous NK cells, they do not express Fc receptors, such as CD-16 receptors, and thus are a less likely target of the VHF virus.

NK-92 cells, methods of modifying NK-92 cells and methods of treating patients with NK-92 cells are disclosed in U.S. Patent No. 8,313,943 and U.S. Patent Application Pub. No. 2014/0099714. Modified NK-92 cells include but are not limited to those described in, *e.g.,* U.S. Patent Nos. 7,618,817, 8,034,332, and 8,313,943, US Patent Application Publication No. 2013/0040386, such as wild type NK-92, NK-92mi and NK-92ci. The NK-92, NK-92mi and NK-92ci cell lines are deposited with the American Type Culture Collection under Deposit Numbers CRL-2407, CRL-2408 and CRL-2409, respectively.

According to the invention, the NK-92 cells are obtained from American Type Culture Collection as Accession No. CRL-2407 or CRL-2408. Preferably, the effective amount of NK-92 cells is between about 1 billion and about 3 billion cells. In one embodiment, the NK-92 cells are inactivated prior to administration. In one embodiment, the NK-92 cells are inactivated by irradiation prior to administration. In one embodiment, the NK-92 cells are administered within the incubation period of the virus (e.g., immediately after exposure or shortly thereafter). In some embodiments, the NK-92 cells are administered prior to 22 days post viral exposure or prior to the manifestation of symptoms of the viral infection. In a further embodiment, the NK-92 cells are administered between 2 to 21 days post viral exposure, or more suitably 4 to 10 days post viral exposure. Thus, in one embodiment, the patient is asymptomatic or does not exhibit symptoms of a viral infection. In one embodiment, the NK-92 cells are administered after the patient has begun to experience one or more symptoms of infection. In one embodiment, the NK-92 cells are administered at a later stage of viral progression, *e*.*g*., when the patient has begun to exhibit multiple symptoms of infection or during the hemorrhagic stage of infection.

In one embodiment, at least one antibody specific for the virus is administered prior to, concurrently with, and/or after administration of the NK-92 cells. In one embodiment, the at least one antibody is a human antibody. In one embodiment, the NK-92 cells and the antibody are administered concurrently. In one embodiment, the NK-92 cells and the antibody are administered sequentially. In a preferred embodiment, the NK-92 cells have not been modified to express an Fc receptor and display it on the cell surface.

In one embodiment, the invention provides NK-92 cells for use in a method for treating viral hemorrhagic fever caused by Ebola virus, wherein the method further comprises administering to the patient serum from a second patient who is recovered or recovering from the viral hemorrhagic fever.

In one embodiment, the NK-92 cells for use in a method further comprises administering to the patient an antiviral drug. In one embodiment, the antiviral drug is ribavirin, taribavirin or brincidofovir. In one embodiment, the antiviral drug is an antisense molecule, double-stranded RNA (RNAi) or morpholino. In one embodiment, the method further comprises administering antibody therapy to the patient. In one embodiment, the antibody is ZMapp or Zmab.

It is described that viral hemorrhagic fever can be caused by a virus selected from the group consisting of an *Arenaviridae,* a *Filoviridae,* a *Bunyaviridae,* a *Flaviviridae,* and a *Rhabdoviridae.* Examples of virus are Ebola or Marburg virus. According to the invention, the virus is Ebola virus. In a preferred embodiment, the virus is Zaire ebolavirus or Sudan ebolavirus. In a preferred embodiment, the Ebola virus causes severe VHF.

In one embodiment, the hemorrhagic disease is mediated by binding to a Fc receptor. In a preferred embodiment, the Fc receptor is CD-16.

### BRIEF DESCRIPTION OF THE DRAWINGS

Figure 1 shows the overall design of the *in vitro* studies for evaluating the therapeutic efficacy of NK-92 cells in Vero cell cultures infected with the Zaire ebolavirus.
Figure 2 shows the yield of the Zaire ebolavirus in different treatment groups over time.

### DETAILED DESCRIPTION OF THE INVENTION

The present invention is limited by the terms of the appended claims, along with the full scope of equivalents to which such claims are entitled.

Particular uses, methods, reagents, compounds, compositions or biological systems can, of course, vary.

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, *etc.* As a non-limiting example, each range discussed herein can be readily broken down into a lower third, middle third and upper third, *etc.* As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

Unless defined otherwise, all technical and scientific terms used herein have the same meaning as commonly understood by one of ordinary skill in the art to which this invention belongs.

In this specification and in the claims that follow, reference will be made to a number of terms that shall be defined to have the following meanings:

The terminology used herein is for the purpose of describing particular embodiments only. As used herein, the singular forms "a", "an" and "the" are intended to include the plural forms as well, unless the context clearly indicates otherwise.

As used herein, the term "about" means that a value may vary +/- 15%, +/-10% or +/- 5% and remain within the scope of the invention. For example, "an IL-2 concentration of about 200 IU/mL" encompasses an IL-2 concentration between 170 IU/mL and 230 IU/mL.

As used herein, the "administration" of an agent to a subject includes any route of introducing or delivering the agent to a subject to perform its intended function. Administration can be carried out by any suitable route, including intravenously, intramuscularly, intraperitoneally, or subcutaneously. Administration includes self-administration and the administration by another.

The term "comprising" is intended to mean that the compositions and methods described herein include the recited elements, but not excluding others. "Consisting essentially of' when used to define compositions and methods, shall mean excluding other elements of any essential significance to the combination. For example, a composition consisting essentially of the elements as defined herein would not exclude other elements that do not materially affect the basic and novel characteristic(s) of the claimed invention. "Consisting of' shall mean excluding more than trace amount of other ingredients and substantial method steps recited. Embodiments defined by each of these transition terms are within the scope of this invention.

As used herein, "concurrent" administration refers to the administration of at least two agents at the same time or at approximately the same time.

As used herein, a "control" is an alternative sample used in an experiment for comparison purpose. A control can be "positive" or "negative." For example, where the purpose of the experiment is to determine a correlation of the efficacy of a therapeutic agent for the treatment for a particular type of disease, a positive control (a composition known to exhibit the desired therapeutic effect) and a negative control (a subject or a sample that does not receive the therapy or receives a placebo) are typically employed.

As used herein, the term "effective amount" refers to a quantity of an agent sufficient to achieve a desired therapeutic effect, *e.g.,* an amount which results in the amelioration of viral hemorrhagic fever or one or more symptoms associated with viral hemorrhagic fever. In the context of therapeutic applications, the amount of NK-92 cells administered to the subject will depend on the type and severity of the viral hemorrhagic fever and on the characteristics of the individual, such as general health, age, sex, body weight and tolerance to drugs. It will also depend on the degree, severity and type of disease. The skilled artisan will be able to determine appropriate dosages depending on these and other factors. NK-92 cells can also be administered in combination with one or more additional therapeutic compounds. In the methods described herein, NK-92 cells may be administered to a subject having one or more signs or symptoms of viral hemorrhagic fever. For example, a "therapeutically effective amount" of NK-92 cells refers to levels at which the physiological effects of viral hemorrhagic fever are, at a minimum, ameliorated. A therapeutically effective amount can be given in one or more administrations. In some embodiments, signs, symptoms or complications of viral hemorrhagic fever include, but are not limited to: generalized fluid distribution problems, hypotension, coagulation disorders, variable degrees of hemorrhage, and widespread focal tissue destruction.

As used herein, the term "expression" refers to the process by which polynucleotides are transcribed into mRNA and/or the process by which the transcribed mRNA is subsequently being translated into peptides, polypeptides, or proteins. If the polynucleotide is derived from genomic DNA, expression may include splicing of the mRNA in a eukaryotic cell. The expression level of a gene may be determined by measuring the amount of mRNA or protein in a cell or tissue sample. In one aspect, the expression level of a gene from one sample may be directly compared to the expression level of that gene from a control or reference sample. In another aspect, the expression level of a gene from one sample may be directly compared to the expression level of that gene from the same sample following administration of NK-92 cells.

As used to describe the present invention, "immunotherapy" refers to any antibody or naturally occurring or modified NK cell or T-cell whether alone or in combination and which are capable of inducing cytotoxicity when contacting an infected cell.

The term "kill" with respect to a cell/cell population is directed to include any type of manipulation that will lead to the death of that cell/cell population.

As used to describe the present invention, "natural killer (NK) cells" are cells of the immune system that kill target cells in the absence of a specific antigenic stimulus, and without restriction according to MHC class. Target cells may be tumor cells or cells harboring viruses. NK cells are characterized by the presence of CD56 and the absence of CD3 surface markers.

The term "endogenous NK cells" is used to refer to NK cells derived from a donor (or the patient), as distinguished from the NK-92 cell line. Endogenous NK cells are generally heterogeneous populations of cells within which NK cells have been enriched. Endogenous NK cells may be intended for autologous or allogeneic treatment of a patient.

"NK-92 cells" refer to the immortal NK cell line, NK-92, which was originally obtained from a patient having non-Hodgkin's lymphoma. For purposes of this disclosure and unless indicated otherwise, the term "NK-92" is intended to refer to the original NK-92 cell lines as well as NK-92 cell lines that have been modified (e.g., by introduction of exogenous genes). NK-92 cells and exemplary and non-limiting modifications thereof are described in U.S. Patent Nos. 7,618,817; 8,034,332; and 8,313,943.

As used herein, "non-irradiated NK-92 cells" are NK-92 cells that have not been irradiated. Irradiation renders the cells incapable of growth and proliferation. It is envisioned that the NK-92 cells will be irradiated at the treatment facility or some other point prior to treatment of a patient, since the time between irradiation and infusion should be no longer than four hours in order to preserve optimal activity. Alternatively, NK-92 cells may be inactivated by another mechanism.

As used to describe the present invention, "inactivation" of the NK-92 cells renders them incapable of growth. Inactivation may also relate to the death of the NK-92 cells. It is envisioned that the NK-92 cells may be inactivated after they have effectively purged an *ex vivo* sample of cells related to a pathology in a therapeutic application, or after they have resided within the body of a mammal a sufficient period of time to effectively kill many or all target cells residing within the body. Inactivation may be induced, by way of non-limiting example, by administering an inactivating agent to which the NK-92 cells are sensitive.

Chimeric receptors generally comprise an exogenous antibody to specific antigen on the target cell surface and an activation/stimulation domain. The term "chimeric antigen receptor" (CAR), as used herein, refers to an extracellular antigen-binding domain that is fused to an intracellular signaling domain of a cell, such as a T cell or a NK-92 cell.

As used to describe the present invention, the terms "cytotoxic" and "cytolytic", when used to describe the activity of effector cells such as NK cells, are intended to be synonymous. In general, cytotoxic activity relates to killing of target cells by any of a variety of biological, biochemical, or biophysical mechanisms. Cytolysis refers more specifically to activity in which the effector lyses the plasma membrane of the target cell, thereby destroying its physical integrity. This results in the killing of the target cell. Without wishing to be bound by theory, it is believed that the cytotoxic effect of NK cells is due to cytolysis.

"Optional" or "optionally" means that the subsequently described event or circumstance can or cannot occur, and that the description includes instances where the event or circumstance occurs and instances where it does not.

As used herein, the terms "multiplicity of infection" or "MOI" refer to the ratio of infectious agents *(e.g.,* a virus) to infection targets *(e.g.,* a cell). For example, when referring to a group of cells inoculated with virus particles, the multiplicity of infection or MOI is the ratio of the number of virus particles to the number of target cells present in a defined space.

The terms "patient," "subject," "individual," and the like are used interchangeably herein, and refer to any animal, or cells thereof whether *in vitro* or *in situ,* amenable to the methods described herein. In a preferred embodiment, the patient, subject, or individual is a mammal. In a particularly preferred embodiment, the patient, subject or individual is a human.

"Plaque forming unit" (PFU) is a functional measurement of the number of particles (e.g., virus particles) capable of forming plaques per unit volume. For example, a solution of Ebola virus with a concentration of 1,000 PFU/µL indicates that 1 µL of the solution contains enough virus particles to produce 1000 infectious plaques in a cell monolayer.

"Treating" or "treatment" as used herein covers the treatment of a disease in a subject, such as a human, and includes: (i) inhibiting a disease, *i.e.*, arresting its development; (ii) relieving a disease, *i.e.*, causing regression of the disease; (iii) slowing progression of the disease; and/or (iv) inhibiting, relieving, or slowing progression of one or more symptoms of the disease.

The term "therapeutic" as used herein means a treatment and/or prophylaxis. A therapeutic effect is obtained by suppression, remission, or eradication of a disease state.
Additionally, some terms used in this specification are more specifically defined below.

### Viral Hemorrhagic Fevers

The invention relates to the treatment of viral hemorrhagic fever caused by Ebola virus.

Viral hemorrhagic fevers (VHFs) are a group of febrile illnesses caused by RNA viruses from several viral families: *Arenaviridae, Filoviridae, Bunyaviridae, Flaviviridae,* and *Rhabdoviridae.* These highly infectious viruses cause a potentially lethal clinical syndrome characterized by fever, malaise, vomiting, mucosal and gastrointestinal (GI) bleeding, edema, and hypotension.

The primary defect in patients with VHF is that of increased vascular permeability. Hemorrhagic fever viruses have an affinity for the vascular system, leading initially to signs such as flushing, conjunctival injection, and petechial hemorrhages, usually associated with fever and myalgias. Later, frank mucous membrane hemorrhage may occur, with accompanying hypotension, shock, and circulatory collapse. The relative severity of the clinical presentation may vary depending on the virus in question, amount, and route of exposure.

In acute disease, patients are extremely viremic, and show elevated levels of multiple cytokines. These cytokines lead to shock and increased vascular permeability, the basic pathophysiologic processes most often seen in viral hemorrhagic fever infection. Another prominent pathologic feature is pronounced macrophage involvement. Inadequate or delayed immune response to these novel viral antigens may lead to rapid development of overwhelming viremia. Extensive infection and necrosis of affected organs can also occur. Hemorrhagic complications are multifactorial and are related to hepatic damage, consumptive coagulopathy, and primary marrow injury to megakaryocytes.

### NK-92 Cells

The invention relates to NK-92 cells from American Type Culture Collection Accession No. CRL-2407 or CRL-2408, wherein the NK-92 cells have not been modified to express an Fc receptor and display it on the cell surface.

The NK-92 cell line is a unique cell line that was discovered to proliferate in the presence of interleukin 2 (IL-2). Gong et al., Leukemia 8:652-658 (1994). These cells have high cytolytic activity against a variety of cancers. The NK-92 cell line is a homogeneous cancerous NK cell population having broad anti-tumor cytotoxicity with predictable yield after expansion. Phase I clinical trials have confirmed its safety profile.

The NK-92 cell line is found to exhibit the CD56^{bright}, CD2, CD7, CD11a, CD28, CD45, and CD54 surface markers. It furthermore does not display the CD1, CD3, CD4, CD5, CD8, CD10, CD14, CD16, CD19, CD20, CD23, and CD34 markers. Growth of NK-92 cells in culture is dependent upon the presence of recombinant interleukin 2 (rIL-2), with a dose as low as 1 IU/mL being sufficient to maintain proliferation. IL-7 and IL-12 do not support long-term growth, nor do other cytokines tested, including IL-1α, IL-6, tumor necrosis factor α, interferon α, and interferon γ. NK-92 has high cytotoxicity even at a low effector:target (E:T) ratio of 1:1. Gong, *et al*., *supra.* NK-92 cells are deposited with the American Type Culture Collection (ATCC), designation CRL-2407.

Heretofore, studies on endogenous NK cells have indicated that IL-2 (1000 IU/mL) is critical for NK cell activation during shipment, but that the cells need not be maintained at 37 °C and 5% carbon dioxide. Koepsell, et al., Transfusion 53:398-403 (2013). However, endogenous NK cells are significantly different from NK-92 cells, in large part because of their distinct origins: NK-92 is a cancer-derived cell line, whereas endogenous NK cells are harvested from a donor (or the patient) and processed for infusion into a patient. Endogenous NK cell preparations are heterogeneous cell populations, whereas NK-92 cells are a homogeneous, clonal cell line. NK-92 cells readily proliferate in culture while maintaining cytotoxicity, whereas endogenous NK cells do not. In addition, an endogenous heterogeneous population of NK cells does not aggregate at high density. Furthermore, endogenous NK cells express Fc receptors, including CD-16 receptor, that are not expressed by NK-92 cells. Fc receptors and particularly CD-16 are contemplated to render endogenous NK cells targets for infection by the virus responsible for VHF in the patient. NK-92 cells do not express CD-16 receptors and are less susceptible to infection by the virus.

### Medical uses

Any references in the description to methods of treatment refer to the compounds, pharmaceutical compositions and medicaments of the present invention for use in a method for treatment of the human (or animal) body by therapy.

NK-92 cells are provided for use in a method for treating viral hemorrhagic fever caused by Ebola virus, the method comprising administering the NK-92 cells to a patient who has or is suspected of having viral hemorrhagic fever. In a preferred embodiment, NK-92 cells are administered intravenously.

In some embodiments, NK-92 cells are administered in a composition comprising NK-92 cells and a medium, such as human serum or an equivalent thereof. In some embodiments, the medium comprises human serum albumin. In some embodiments, the medium comprises human plasma. In some embodiments, the medium comprises about 1% to about 15% human serum or human serum equivalent. In some embodiments, the medium comprises about 1% to about 10% human serum or human serum equivalent. In some embodiments, the medium comprises about 1% to about 5% human serum or human serum equivalent. In a preferred embodiment, the medium comprises about 2.5% human serum or human serum equivalent. In some embodiments, the serum is human AB serum. In some embodiments, a serum substitute that is acceptable for use in human therapeutics is used instead of human serum. Such serum substitutes may be known in the art, or developed in the future. Although concentrations of human serum over 15% can be used, it is contemplated that concentrations greater than about 5% will be cost-prohibitive. In some embodiments, NK-92 cells are administered in a composition comprising NK-92 cells and an isotonic liquid solution that supports cell viability. In some embodiments, NK-92 cells are administered in a composition that has been reconstituted from a cryopreserved sample.

Expression of a cytokine (such as interleukin-2) can also correlate with viability or cytotoxicity of the modified NK-92 cells.

NK-92 cells can be administered to an individual in need of treatment by absolute numbers of cells, *e.g.,* said individual can be administered from about 1000 cells/injection to up to about 10 billion cells/injection, such as at about, at least about, or at most about, 1×10⁸, 1×10⁷, 5×10⁷, 1×10⁶, 5×10⁶, 1×10⁵, 5×10⁵, 1×10⁴, 5×10⁴, 1×10³, 5×10³ (and so forth) NK-92 cells per injection, or any ranges between any two of the numbers, end points inclusive. In other embodiments, NK-92 cells can be administered to an individual in need of treatment by relative numbers of cells, *e.g.,* said individual can be administered about 1000 cells to up to about 10 billion cells per kilogram of the individual, such as at about, at least about, or at most about, 1×10⁸, 1×10⁷, 5×10⁷, 1×10⁶, 5×10⁶, 1×10⁵, 5×10⁵, 1×10⁴, 5×10⁴, 1×10³, 5×10³ (and so forth) NK-92 cells per kilogram of the individual, or any ranges between any two of the numbers, end points inclusive. In a preferred embodiment, between about 1 billion and about 3 billion NK-92 cells are administered to a patient.

The NK-92 cells, and optionally antibody and/or other anti-viral agents as described below, can be administered once to a patient infected with a virus or can be administered multiple times, *e*.*g*., once every 1, 2, 3, 4, 5, 6, 7, 8, 9, 10, 11, 12, 13, 14, 15, 16, 17, 18, 19, 20, 21, 22 or 23 hours, or once every 1, 2, 3, 4, 5, 6 or 7 days, or once every 1, 2, 3, 4, 5, 6, 7, 8, 9, 10 or more weeks during therapy, or any ranges between any two of the numbers, end points inclusive.

The invention relates to viral hemorrhagic fever caused by Ebola virus. More generally, VHF may be caused by a virus from one of the following families: *Arenaviridae,* a *Bunyaviridae,* a *Filoviridae,* a *Flaviviridae,* and a *Rhabdoviridae.* The family *Arenaviridae* includes viruses that cause Lassa fever, Lujo virus, Argentine hemorrhagic fever, Bolivian hemorrhagic fever, Brazilian hemorrhagic fever, and Venezuelan hemorrhagic fever. The family *Bunyaviridae* includes viruses that cause hemorrhagic fever with renal syndrome (HFRS), Crimean-Congo hemorrhagic fever (CCHF), Garissa virus, Ilesha virus, and Rift Valley fever (RVF). The family *Filoviridae* includes Ebola and Marburg. There are five known strains of ebolavirus, although the Zaire ebolavirus and Sudan ebolavirus strains have thus far been the most infective in human populations. The family *Flaviviridae* includes dengue fever, yellow fever, Omsk hemorrhagic fever virus and Kyasanur Forest disease virus. The family *Rhabdoviridae* includes rabies and Bas-Congo Virus. In one embodiment, the NK-92 cells are inactivated prior to administration. In one embodiment, the NK-92 cells are inactivated by irradiation prior to administration. In one embodiment, the NK-92 cells are administered within the incubation period of the virus (*e*.*g*., immediately after exposure or shortly thereafter). In some embodiments, the incubation period of the virus is less than 22 days or prior to the manifestation of symptoms of the viral infection. In a further embodiment, the incubation period of the virus is between 2 to 21 days, or more suitably 4 to 10 days. Thus, in one embodiment, the patient is asymptomatic or does not exhibit symptoms of a viral infection. In one embodiment, the NK-92 cells are administered after the patient has begun to experience one or more symptoms of infection. In one embodiment, the NK-92 cells are administered at a later stage of viral progression, *e*.*g*., when the patient has begun to exhibit multiple symptoms of infection or during the hemorrhagic stage of infection.

### Combination Treatment

In one embodiment, the NK-92 cells are for use in a method of treatment wherein the NK-92 cells are administered to the patient in conjunction with one or more treatments for the virus being treated. Without being bound by theory, it is believed that co-treatment of a patient with NK-92 cells and another therapy for the virus will allow the NK-92 cells and the alternative therapy to give the endogenous immune system a chance to clear the virus that heretofore had overwhelmed such endogenous action.

Virus-specific antibodies bind to particular protein antigens that are uniquely expressed on the surfaces of virus-infected cells. NK-92 cells can be modified such that an antibody is associated with the NK-92 cell surface. In a preferred embodiment, the antibody is specific for the VHF-causing virus. In this way, the NK-92 cell can be specifically targeted to the virus or cells expressing viral proteins. Alternatively, the antibody can be administered in conjunction with administration of NK-92 cells. An antibody specific for the virus to be treated can be administered prior to, concurrently with, and/or after administration of the NK-92 cells. In one embodiment, an anti-Ebola antibody described in Maruyama, et al., Journal of Infectious Diseases 1999; 179(Suppl 1):S235-9, can be administered prior to, concurrently with, and/or after administration of the NK-92 cells. In one embodiment, an anti-Ebola antibody described in WO 2013/095738, Qiu X et al., Nature 514(7520):47-53 (2014), or Qiu X et al., Sci Rep 3:3365 (2013), can be administered prior to, concurrently with, and/or after administration of the NK-92 cells. In one embodiment, the antibody is ZMapp or Zmab.

Antibodies to a virus that causes VHF can be produced by any method. For example, antibodies can be produced by obtaining B cells, bone marrow, or other samples from previously one or more patients who were infected by the virus and recovered or were recovering when the sample was taken. Methods of identifying, screening, and growing antibodies (e.g., monoclonal antibodies) from these samples are known. For example, a phage display library can be made by isolating RNA from the sample or cells of interest, preparing cDNA from the isolated RNA, enriching the cDNA for heavy-chain and/or light-chain cDNA, and creating libraries using a phage display vector. Libraries can be prepared and screened as described, for example, in Maruyama, *et al..* Antibodies can be made by recombinant methods or any other method. Isolation, screening, characterization, and production of human monoclonal antibodies is also described in Beerli, et al., PNAS 2008; 105(38):14336-14341.

In one aspect, an agent that disrupts the IFN-I pathway (*e*.*g*., an antibody specific for CD8⁺ T-cells, IFN-I, or IFN-I receptors) is administered. In one embodiment, the agent is administered independently of administration of NK-92 cells. In one embodiment, the agent is administered in conjunction with NK-92 cells.

In one embodiment, serum, plasma, and/or immune cells *(e.g.,* B cells, T cell and the like) from a second patient who is recovered or recovering from the viral hemorrhagic fever are also administered to the patient. Without being bound by theory, it is believed that administration of serum, plasma, and/or immune cells from a previously infected (but recovered or recovering) patient (also called convalescent or convalescent-phase serum) provides antibodies to the virus that aid in targeting of the virus or virus-infected cells by the patient's immune system and/or the exogenous NK-92 cells.

In one embodiment, a bispecific antibody can be used that binds the virus or virus-infected cell and also binds a cell-surface protein present on the surface of NK-92 cells. In a preferred embodiment, the NK-92 cells are not modified to express an Fc receptor. For example, the bispecific antibody induces antibody-dependent cell-mediated cytotoxicity (ADCC) by binding to both the virus-infected cell and the NK-92 cell, independently of an Fc receptor.

In one embodiment, the patient is administered an antiviral drug in combination with NK-92 cells and/or antibody. In one embodiment, the antiviral drug is ribavirin, taribavirin, or brincidofovir. In one embodiment, the antiviral drug is an antisense molecule, double-stranded RNA (RNAi) or morpholino. Antisense molecules are segments of DNA or RNA that are designed as complementary molecule to critical sections of viral genomes. Binding of these antisense segments to target sections of the genome blocks the operation of those genomes.

In one embodiment, an antibody to the virus and/or antiviral drug is administered to the patient in conjunction with the NK-92 cells. In one embodiment, the NK-92 cells and antibody and/or antiviral drug are administered to the patient together, *e.g.,* in the same formulation; separately, *e.g.,* in separate formulations, concurrently; or can be administered separately, *e*.*g*., on different dosing schedules or at different times of the day. When administered separately, the antiviral drug and/or antibody can be administered in any suitable route, such as intravenous or oral administration.

### EXAMPLES

The present invention is further illustrated by the following examples.

### Example 1: Effect of NK-92 Cells on Vero Cells Infected with Zaire Ebolavirus

This Example demonstrates the therapeutic effect of NK-92 cells on Vero cells infected with Zaire ebolavirus.

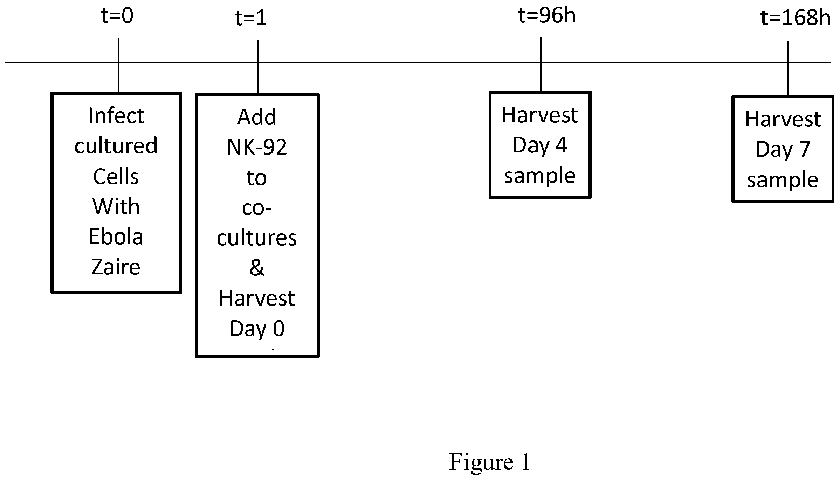

*Experimental Design.* Figure 1 illustrates the general protocol of the viral infection assays in Vero-E6 (African green monkey kidney) cell cultures. Monolayers of the Vero-E6 cell line were infected with Zaire ebolavirus (MOI =1). One hour post-infection, NK-92 cells were added to the infected Vero cells at one of the following concentrations: 1× 10⁵ , 5× 10⁵, 1× 10⁶. Supernatants for the Vero-E6 + NK-92 co-culture and Vero-E6 control monoculture were collected on the day of infection (Day 0), 96 hours post infection and 168 hours post infection (Day 7). Each experimental condition was tested in triplicate and the virus yield for each replicate was determined by standard virus plaque assay. *See* Jahrling et al., Arch. Virol. Suppl. 11:135-140 (1996); Bray et al., J. Infect. Dis. 179:S248-S258 (1999). NK-92 cells exposed to the Zaire ebolavirus served as a control for the infection assay at all the experimental time points.

| | **1×10⁶ NK-92 (control)** | | **Infected Vero only** | | **Infected Vero 1× 10⁵ NK-92** | | **Infected Vero 5x 10⁵ NK-92** | | **Infected Vero 1× 10⁶ NK-92** | |
|---|---|---|---|---|---|---|---|---|---|---|
| | **Avg. Yield** | **Std. Dev** | **Avg. Yield** | **Std. Dev** | **Avg. Yield** | **Std. Dev** | **Avg. Yield** | **Std. Dev** | **Avg. Yield** | **Std. Dev** |
| **Day 0** | 3.80 E+03 | 2.70 E+02 | 9.85 E+03 | 1.16 E+04 | 3.23 E+03 | 3.03 E+02 | 7.34 E+03 | 7.95 E+03 | 3.09 E+03 | 1.03 E+02 |
| **Day 4** | 1.26 E+04 | 7.17 E+03 | 4.26 E+06 | 3.51 E+05 | 5.50 E+06 | 3.14 E+06 | 5.20 E+05 | 2.00 E+05 | 4.27 E+04 | 3.35 E+04 |
| **Day 7** | 1.79 E+03 | 3.12 E+02 | 7.29 E+06 | 3.29 E+06 | 1.75 E+06 | 3.47 E+05 | 1.48 E+05 | 6.95 E+04 | 4.83 E+03 | 1.63 E+03 |

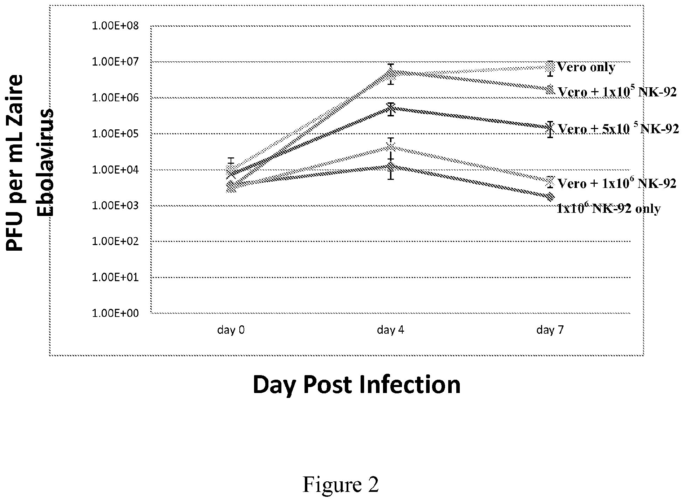

*Results.* The average virus yield for each treatment group is described in Table 1 and Figure 2. Zaire ebolavirus replicates to an average titer of approximately 4.2 × 10⁶/ml by day 4 and 7.3 × 10⁶/ml by day 7 under monoculture conditions in permissive Vero cells, while titers in NK-92 monocultures remain near input virus levels. As shown in Figure 2, NK-92 cells that were exposed to Zaire ebolavirus showed little to no increase in virus yield over 7 days, thus demonstrating that NK-92 cells are not vulnerable to ebolavirus infection. In contrast, infected Vero cells exhibited high virus yields that exceeded 1.00 × 10⁶ PFU/mL at days 4 and 7. Figure 2 also demonstrates that NK-92 cells suppressed ebolavirus-induced plaque formation in a dose-dependent manner (>3 orders of magnitude difference between the infected Vero compared to the infected Vero + high dose (1 × 10⁶) NK-92 group).

These results demonstrate that NK-92 cells inhibit Zaire ebolavirus-induced infection. Accordingly, NK-92 cells are useful in methods for treating viral hemorrhagic fever caused by Ebola.

### Example 2: Effect of NK-92 Cells on Vero Cells Infected with Sudan Ebolavirus

This Example demonstrates the therapeutic effect of NK-92 cells on Vero cells infected with Sudan ebolavirus.

*Experimental Design.* Vero-E6 cells are plated in tissue culture dishes at 7.5-9.0×10⁵ cells/well to achieve the desired % confluency. Monolayers of the Vero cell line will be infected with Sudan ebolavirus (MOI =0.1). The cells are incubated with virus inoculums for 24 hours. On the following day, Gibco^{®} RPMI 1640 medium supplemented with 20% Fetal Bovine Serum and IL-2 will be added to each well. NK-92 cells are then added to the infected Vero cells at one of the following concentrations: 1× 10⁵, 5× 10⁵, 1× 10⁶. Cell supernatant will be harvested on the day of infection (Day 0), 96 hours post infection and 168 hours post infection (Day 7). Each experimental condition will be tested in triplicate and the virus yield for each replicate will be determined by standard virus plaque assay. *See* Jahrling et al., Arch. Virol. Suppl. 11:135-140 (1996); Bray et al., J. Infect. Dis. 179:S248-S258 (1999). NK-92 cells exposed to the Sudan ebolavirus serves as a control for the infection assay at all the experimental time points.

*Results.* NK-92 cells that are exposed to Sudan ebolavirus show no increase in virus yield over 7 days. Infected Vero cells show a dramatic increase in plaque formation over time. NK-92 cells suppress ebolavirus-induced plaque formation in a dose-dependent manner.

These results will demonstrate that NK-92 cells inhibit Sudan ebolavirus-induced infection. Accordingly, NK-92 cells are useful in methods for treating viral hemorrhagic fever caused by Ebola.

### Example 3: Reversion of Advanced Ebola Virus Disease in Primates with NK-92 Cells

This Example demonstrates the therapeutic efficacy of NK-92 cells in mammalian subjects. Twenty male rhesus macaques, ranging from 2.5 to 3.5 kg (2 years old) will be randomized into treatment or placebo groups. Animals are fed standard monkey chow, fruits, and vegetables. All animals are challenged intramuscularly with Zaire ebolavirus (titer: 628 PFU). Administration of the first dose of NK-92 cells or placebo is initiated at 3 days post infection (dpi), with two additional identical doses spaced 3 days apart. Animals are scored daily for signs of disease, in addition to changes in food and water consumption. The following clinical parameters are evaluated at 14, 21, and 28 dpi: blood for serum biochemistry, cell counts and viremia.

*Blood counts and blood biochemistry.* Complete blood counts are performed with the VetScan HM5 (Abaxis Veterinary Diagnostics). The following parameters will be evaluated: levels of white blood cells, lymphocytes, percentage of lymphocytes, levels of platelets, neutrophils and percentage of neutrophils. Blood biochemistry is performed with the VetScan VS2 (Abaxis Veterinary Diagnostics). The following parameters will be evaluated: levels of alkaline phosphatase, alanine aminotransferase, blood urea nitrogen, creatinine, and total bilirubin.

*Ebolavirus titration by tissue culture infectious dose.* Titration of live ebolavirus is determined by adding tenfold serial dilutions of whole blood to Vero E6 cells, with three replicates per dilution. The plates are scored for cytopathic effect (plaque formation) at 14 dpi, and viral titers are calculated.

*Results.* It is anticipated that the animals within the placebo group will experience high fever, viremia and abnormalities in blood count and blood chemistry. In contrast, animals treated with NK-92 cells will experience partial or complete amelioration of one or more clinical symptoms associated with ebolavirus infection.

Accordingly, NK-92 cells are useful in methods for treating viral hemorrhagic fever caused by Ebola.

## Claims

1. NK-92 cells for use in a method for treating viral hemorrhagic fever caused by Ebola virus, the method comprising administering the NK-92 cells to a patient who has or is suspected of having viral hemorrhagic fever, wherein the NK-92 cells are from American Type Culture Collection Accession No. CRL-2407 or CRL-2408 and wherein the NK-92 cells have not been modified to express an Fc receptor and display it on the cell surface.

2. The NK-92 cells for use according to claim 1, wherein between about 1 billion and about 3 billion NK-92 cells are administered.

3. The NK-92 cells for use according to claim 1 or claim 2, wherein the NK-92 cells are irradiated prior to administration.

4. The NK-92 cells for use according to any one of claims 1-3, wherein the method further comprises administering at least one antibody specific for the Ebola virus causing the viral hemorrhagic fever.

5. The NK-92 cells for use according to claim 4, wherein the NK-92 cells and the antibody are administered concurrently.

6. The NK-92 cells for use according to claim 4, wherein the NK-92 cells and the antibody are administered sequentially.

7. The NK-92 cells for use according to any one of claims 1-6, wherein the method further comprises administering to the patient serum from a second patient who is recovered or recovering from the viral hemorrhagic fever.

8. The NK-92 cells for use according to any of claims 1 to 7, wherein the Ebola virus is Sudan ebolavirus or Zaire ebolavirus.

9. The NK-92 cells for use according to any one of claims 1-8, further comprising administering to the patient an antiviral drug, optionally wherein the antiviral drug is ribavirin or taribavirin.

## Patentansprüche

1. NK-92-Zellen zur Verwendung in einem Verfahren zum Behandeln von viralem hämorrhagischem Fieber, das durch Ebola-Virus verursacht wird, wobei das Verfahren das Verabreichen der NK-92-Zellen an einen Patienten umfasst, der virales hämorrhagisches Fieber aufweist oder bei dem der Verdacht besteht, dass er dieses aufweist, wobei die NK-92-Zellen von der American Type Culture Collection, ZugangsNr. CRL-2407 oder CRL-2408 sind und wobei die NK-92-Zellen nicht modifiziert worden sind, um einen Fc-Rezeptor zu exprimieren und ihn auf der Zelloberfläche anzuzeigen.

2. NK-92-Zellen zur Verwendung nach Anspruch 1, wobei zwischen etwa 1 Milliarde und etwa 3 Milliarden NK-92-Zellen verabreicht werden.

3. NK-92-Zellen zur Verwendung nach Anspruch 1 oder Anspruch 2, wobei die NK-92-Zellen vor Verabreichung bestrahlt werden.

4. NK-92-Zellen zur Verwendung nach einem der Ansprüche 1-3, wobei das Verfahren ferner das Verabreichen von zumindest einem Antikörper umfasst, der spezifisch für das Ebola-Virus ist, welches das virale hämorrhagische Fieber verursacht.

5. NK-92-Zellen zur Verwendung nach Anspruch 4, wobei die NK-92-Zellen und der Antikörper gleichzeitig verabreicht werden.

6. NK-92-Zellen zur Verwendung nach Anspruch 4, wobei die NK-92-Zellen und der Antikörper nacheinander verabreicht werden.

7. NK-92-Zellen zur Verwendung nach einem der Ansprüche 1-6, wobei das Verfahren ferner das Verabreichen von Serum von einem zweiten Patienten, der sich von dem viralen hämorrhagischen Fieber erholt hat oder erholt, an den Patienten umfasst.

8. NK-92-Zellen zur Verwendung nach einem der Ansprüche 1 bis 7, wobei das Ebolavirus Sudan-Ebolavirus oder Zaire-Ebolavirus ist.

9. NK-92-Zellen zur Verwendung nach einem der Ansprüche 1-8, ferner umfassend das Verabreichen eines antiviralen Arzneimittels an den Patienten, wobei optional das antivirale Arzneimittel Ribavirin oder Taribavirin ist.

## Revendications

1. Cellules NK-92 destinées à être utilisées dans une méthode de traitement de la fièvre hémorragique virale provoquée par le virus Ebola, la méthode comprenant l'administration des cellules NK-92 à un patient qui est atteint ou est soupçonné d'être atteint d'une fièvre hémorragique virale, lesdites cellules NK-92 provenant des numéros d'accession collection américaine de cultures types CRL-2407 ou CRL-2408 et lesdites cellules NK-92 n'ayant pas été modifiées pour exprimer un récepteur Fc et le présenter à la surface des cellules.

2. Cellules NK-92 destinées à être utilisées selon la revendication 1, entre environ 1 milliard et environ 3 milliards de cellules NK-92 étant administrées.

3. Cellules NK-92 destinées à être utilisées selon la revendication 1 ou la revendication 2, lesdites cellules NK-92 étant irradiées avant administration.

4. Cellules NK-92 destinées à être utilisées selon l'une quelconque des revendications 1 à 3, ladite méthode comprenant en outre l'administration d'au moins un anticorps spécifique au virus Ebola provoquant la fièvre hémorragique virale.

5. Cellules NK-92 destinées à être utilisées selon la revendication 4, lesdites cellules NK-92 et ledit anticorps étant administrés en même temps.

6. Cellules NK-92 destinées à être utilisées selon la revendication 4, lesdites cellules NK-92 et ledit anticorps étant administrés de manière séquentielle.

7. Cellules NK-92 destinées à être utilisées selon l'une quelconque des revendications 1 à 6, ladite méthode comprenant en outre l'administration au patient d'un sérum provenant d'un second patient qui a guéri ou est en train de guérir de la fièvre hémorragique virale.

8. Cellules NK-92 destinées à être utilisées selon l'une quelconque des revendications 1 à 7, ledit virus Ebola étant le virus Ebola du Soudan ou le virus Ebola du Zaïre.

9. Cellules NK-92 destinées à être utilisées selon l'une quelconque des revendications 1 à 8, comprenant en outre l'administration au patient d'un médicament antiviral, éventuellement ledit médicament antiviral étant la ribavirine ou la taribavirine.
